(19) Europäisches Patentamt
European Patent Office
Office européen des brevets

(11) Veröffentlichungsnummer : **0 386 508 B1**

(12) # EUROPÄISCHE PATENTSCHRIFT

(45) Veröffentlichungstag der Patentschrift : **12.05.93 Patentblatt 93/19**

(51) Int. Cl.$^5$ : **C07C 333/20,** C07C 329/16, A01N 47/06, A01N 47/14

(21) Anmeldenummer : **90103086.6**

(22) Anmeldetag : **17.02.90**

(54) **Quaternäre Ammoniumsalze, Verfahren zu deren Herstellung und Verwendung derselben.**

(30) Priorität : **04.03.89 DE 3907070**

(43) Veröffentlichungstag der Anmeldung : **12.09.90 Patentblatt 90/37**

(45) Bekanntmachung des Hinweises auf die Patenterteilung : **12.05.93 Patentblatt 93/19**

(84) Benannte Vertragsstaaten : **AT BE CH DE FR GB IT LI NL**

(56) Entgegenhaltungen :
**DD-A- 228 162**
**DD-A- 236 669**
**DE-B- 1 921 988**
**US-A- 2 961 466**
**US-A- 3 280 162**
**US-A- 3 407 222**

(73) Patentinhaber : **DEGUSSA AKTIENGESELLSCHAFT**
**Weissfrauenstrasse 9**
**W-6000 Frankfurt 11 (DE)**

(72) Erfinder : **Werle, Peter, Dr.**
**Im Börner 43**
**W-6460 Gelnhausen 2 (DE)**
Erfinder : **Trageser, Martin**
**Leipziger Strasse 14**
**W-6460 Gelnhausen-Hoechst (DE)**
Erfinder : **Weiss, Svea**
**Danziger Strasse 1a**
**W-6054 Rodgau 6 (DE)**

**EP 0 386 508 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue quaternäre Ammoniumsalze der allgemeinen Formel

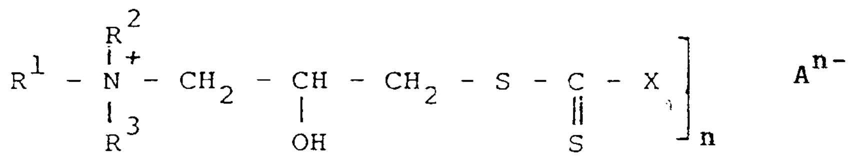

deren Herstellung sowie die Verwendung derselben als Mikrobiozide.

Verschiedene Klassen quaternärer Ammoniumsalze, insbesondere solche, welche mindestens eine ($C_8$-$C_{16}$) -Alkylgruppe aufweisen, finden als Mikrobiozide im Hygienebereich und in der Industrie Anwendung - siehe Seifen-Öle-Fette-Wachse 104 Nr. 15 (1978), 433-479. Die anwendungsspezifischen Erfordernisse sowie Aspekte der Ökologie und Toxikologie und nicht zuletzt der Wirtschaftlichkeit schaffen ein Bedürfnis, die bestehende Palette an quaternären Ammoniumsalzen zu erweitern, weil ausgewählte Produkte im allgemeinen nicht allen Erfordernissen gleichzeitig gerecht werden.

Es ist auch bekannt, daß aus tertiären Aminen wie Dodecyl-dimethylamin und Epichlorhydrin leicht zugängliche quaternäre Ammoniumsalze mit einem 2-Hydroxy-3-chlorpropyl-Substituenten mikrobiozide Aktivität aufweisen. Es zeigte sich allerdings, daß die Wirkung gegenüber Pilzen, wie Aspergillus niger, häufig unbefriedigend ist. Es bestand somit ein Interesse, das Eigenschaftsspektrum dieser Stoffklasse zu verbessern.

Natrium-dithiocarbaminate finden in wäßriger Lösung Verwendung als Mikrobiozide, zum Beispiel zur Schleimbekämpfung in Wasserkreisläufen oder als Konservierungsmittel in Emulsionen und Dispersionen - siehe Firmenprospekt der Firma Bayer AG "Preventol Z" (09/1981). Diese gleichfalls leicht zugängliche und im allgemeinen preiswerte Biozid-Stoffklasse hat aber den Nachteil eines ziemlich begrenzten Wirkungsspektrums.

Es wurde nun gefunden, daß aus quaternären Ammoniumsalzen mit einem am Stickstoff gebundenen 2-Hydroxy-3-chlorpropyl- oder einem 2,3-Epoxypropylrest durch Umsetzung mit einem Alkalisalz einer Dithiocarbaminsäure oder einem Alkalisalz eines Dithiokohlensäure-O-esters neue quaternäre Ammoniumsalze entstehen. Diese neuen Salze zeichnen sich durch eine hohe mikrobiozide Wirkung aus, welche diejenige der Ausgangssubstanzen zum Teil ganz erheblich übertrifft.

Die erfindungsgemäßen quaternären Ammoniumverbindungen haben die allgemeine Formel (I)

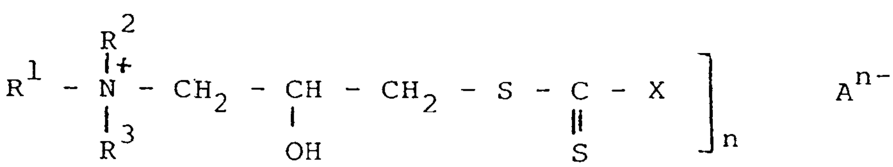 (I)

worin bedeuten:

$R^1$ eine lineare ($C_8$-$C_{16}$)-Alkylgruppe oder eine ($C_8$-$C_{16}$)-Alkylphenoxyethoxyethylgruppe, wenn $R^2$ und $R^3$ gleich sind und Methyl oder Hydroxyethyl bedeuten, oder ein Strukturelement der allgemeinen Formel (II)

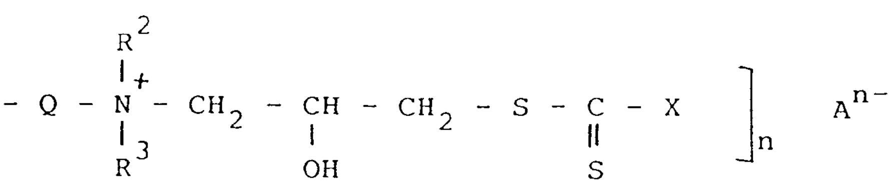 (II),

worin Q für α, ω-($C_2$-$C_6$)-Alkylen, das innenständig auch methylsubstituiert sein kann,

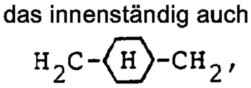

$CH_2$-$CH_2$-O-$CH_2$-$CH_2$ oder $CH_2$-$CH_2$-N($CH_3$)-$CH_2$-$CH_2$ steht, wenn $R^2$ und $R^3$ in Formel (I) und (II) jeweils Methyl bedeuten oder im Falle von Q gleich Ethylen $R^2$ in (I) und (II) Methyl und $R^3$ in (I) und (II) auch gemeinsam für eine Ethylengruppe stehen können;

X die Gruppe $OR^4$, worin $R^4$ für einen linearen oder verzweigten Alkylrest mit 8 bis 18 C-Atomen steht, oder
die Gruppe $NR^5R^6$, worin $R^5$ Wasserstoff oder eine lineare ($C_8$-$C_{18}$)-Alkylgruppe und $R^6$ eine ($C_1$-$C_{18}$)-Alkyl- oder Benzylgruppe bedeuten oder $NR^5R^6$ für den Pyrrolidino-, 1,3-Oxazolidino, Piperidino oder Morpholinorest steht, wobei in das Strukturelement (II) enthaltenden Salzen die Gruppe $NR^5R^6$ mindestens einen linearen ($C_8$-$C_{12}$)-Alkylrest aufweist, oder
ein Strukturelement der allgemeinen Formel (III) oder (IV)

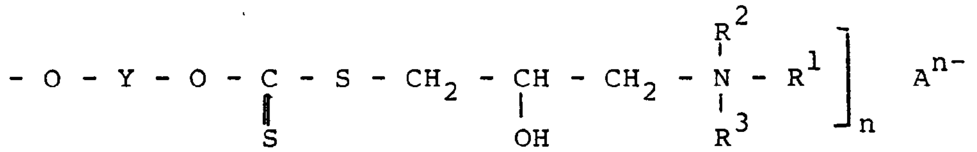
(III)

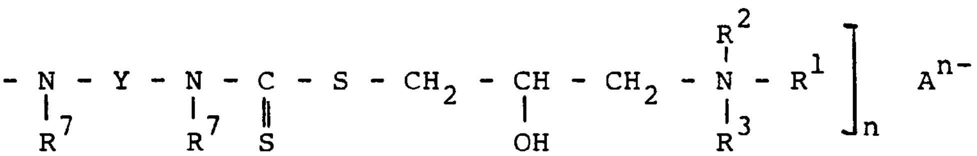
(IV),

worin Y die gleiche Bedeutung wie Q hat und $R^7$ Wasserstoff oder Methyl bedeutet, jedoch im Falle von Y gleich Ethylen beide Substituenten $R^7$ auch gemeinsam für eine Ethylengruppe stehen können, und wobei in das Strukturelement (III) oder IV enthaltenden Salzen $R^1$ nicht dem Strukturelement (II) entspricht;

A ein Halogenid, Sulfat, Hydrogensulfat, Hydrogen- oder Dihydrogenphosphat, Nitrat, Formiat, Acetat, Benzoat und

n die Wertigkeit 1 oder 2 des Anions.

Die Unteransprüche zu diesen neuen quaternären Ammoniumsalzen betreffen Salze mit ausgewählten Substituenten und Strukturelementen. Diese Stoffe zeichnen sich durch ihre besondere biozide Wirksamkeit aus.

Die erfindungsgemäßen quaternären Ammoniumsalze können ein oder zwei Ammoniumkationen enthalten. Salze mit dem Strukturelement der allgemeinen Formel (II), (III) oder (IV) weisen zwei Ammoniumkationen auf; bevorzugt sind hiervon Salze mit dem Strukturelement der Formel (IV) mit Y gleich ($C_2$-$C_6$)-Alkylen und $R^7$ gleich H. Verbindungen mit einem Ammoniumkation entsprechen der allgemeinen Formel (I) mit einem einwertigen Rest für X und $R^1$. Salze mit $R^2$ und $R^3$ gleich Methyl und $R^1$ gleich n-($C_8$-$C_{12}$)-Alkyl und X gleich n-($C_8$-$C_{12}$)-Alkyloxy oder Mono- oder Di-n-($C_8$-$C_{12}$)-Alkylamino oder Methylamino im Falle von $R^1$ gleich Dodecyl sind besonders bevorzugt.

Das bzw. die Anionen der Ammoniumsalze können ein- oder zweiwertig sein. Anionen starker und mittelstarker Säuren werden bevorzugt. Erfindungsgemäße quaternäre Ammoniumchloride sind besonders einfach zugänglich, wenn 2-Hydroxy-3-chlorpropyl-substituierte quaternäre Ammoniumchloride als Ausgangsstoffe dienen.

Mikrobiozid wirksame erfindungsgemäße Verbindungen weisen mindestens eine ($C_8$-$C_{16}$)-, vorzugsweise eine lineare ($C_8$-$C_{12}$)-Alkylgruppe oder eine ($C_8$-$C_{16}$)-Alkylphenoxyethoxyethylgruppe am Ammoniumstickstoff und/oder eine ($C_8$-$C_{16}$)-, vorzugsweise eine lineare ($C_8$-$C_{12}$)-Alkylgruppe als Bestandteil des Dithiocarbamat- bzw. Dithiokohlensäureester-Strukturelements auf.

Die erfindungsgemäßen quaternären Ammoniumsalze der allgemeinen Formel (I) lassen in einfacher Weise dadurch herstellen, daß man in wäßriger Lösung ein Alkalisalz einer Dithiocarbaminsäure der allgemeinen Formel (Va) oder (Vb)

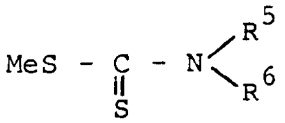 (Va) 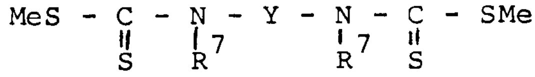 (Vb)

oder ein Alkalisalz eines Dithiokohlensäure-O-esters der allgemeinen Formel (Vc) oder (Vd)

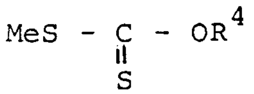 (Vc) 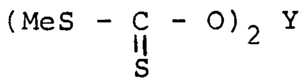 (Vd),

worin Me Li, Na oder K sein kann und $R^4$, $R^5$, $R^6$, $R^7$ und Y die bei den erfindungsgemäßen Stoffen angegebene Bedeutung haben, mit einem 3-Halogen-2-hydroxypropyltrialkylammoniumsalz der allgemeinen Formel (VIa) oder (VIb)

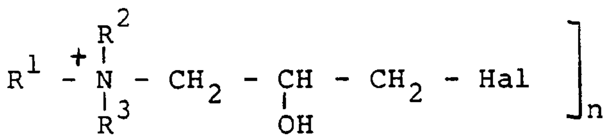 $A^{n-}$ (VIa)

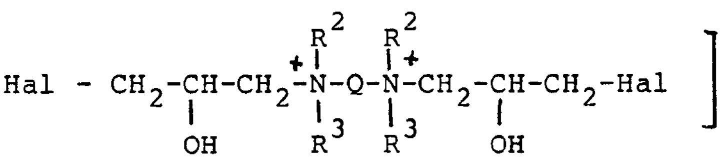 $\frac{2}{n} \cdot A^{n-}$ (VIb),

worin Hal Chlor oder Brom und $R^1$, $R^2$, $R^3$ A, n und Q die bei den erfindungsgemäßen Stoffen angegebene Bedeutung haben, umsetzt und das gebildete Salz der Formel (I) in bekannter Weise aus dem Reaktionsgemisch isoliert und, falls erwünscht, reinigt. Alternativ können die quaternären Ammoniumsalze der Formel (I) auch dadurch hergestellt werden, daß ein Salz der allgemeinen Formel Va, Vb, Vc oder Vd in wäßriger Lösung mit einem 2,3-Epoxypropyl-trialkylammoniumsalz der allgemeinen Formel (VIIa) oder (VIIb)

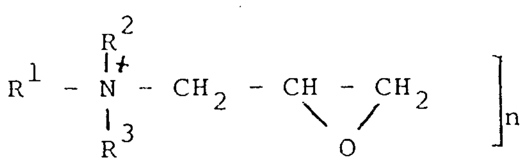 $A^{n-}$ (VIIa)

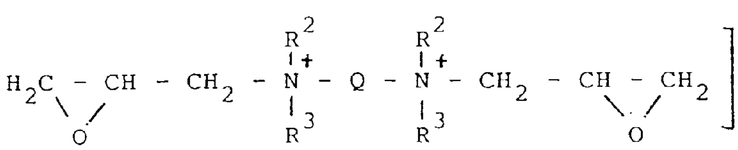 $\frac{2}{n} A^{n-}$ (VIIb),

worin $R^1$, $R^2$, $R^3$, A, n und Q die gleiche Bedeutung wie in Formel (I) und (II) haben, umgesetzt und das sich bildende quaternäre Ammoniumhydroxid durch Zugabe einer äquivalenten Menge einer Säure $H_nA$ in das Salz der Formel (I) überführt wird.

Die Reaktionspartner werden im allgemeinen im äquivalenten Verhältnis miteinander umgesetzt, also im Molverhältnis 1:1, wenn Verbindungen gemäß Formel (Va) oder (Vc) mit solchen der Formel (VIa) mit $R^1$ ungleich dem Strukturelement der Formel (II) umgesetzt werden, und 1:2, wenn anstelle der Verbindungen der Formel (Va) oder (Vc) solche der Formel (Vb) oder (Vd) zum Einsatz kommen. Verbindungen der Formel VIb

werden mit solchen der Formel (Va) oder (Vc) im Molverhältnis 1:2 umgesetzt. Ein Überschuß eines Reaktionspartners stört im allgemeinen nicht und kann dann sogar nützlich sein, wenn das Reaktionsgemisch ohne Isolierung der erfindungsgemäßen Salze als biozides Mittel oder Bestandteil von solchen verwendet wird.

Die Umsetzung erfolgt bei Raumtemperatur, jedoch kann auch erwärmt werden. Besonders leicht gelingt die Umsetzung, wenn A in den Salzen der Formel (VIa) und (VIb) das gleiche Halogenid ist wie dasjenige des 2-Hydroxy-3-halogenpropyl-Substituenten. Die wäßrige Lösung, in welcher die Umsetzung erfolgt, kann auch wasserlösliche organische Lösungsmittel, wie beispielsweise niedere Alkohole oder Aceton enthalten. Bei der Umsetzung in wäßrig-alkoholischer Lösung trennt sich das Salz der Formel I meist als zweite Phase ab und kann dann nach Abtrennung der wäßrigen Phase in bekannter Weise gereinigt werden.

Falls erwünscht, kann das herstellungsbedingt anwesende Anion A der Salze der Formel I in bekannter Weise, beispielsweise unter Verwendung von Ionenaustauschern, gegen andere Anionen A ausgetauscht werden.

Die erfindungsgemäßen Salze der Formel I lassen sich auch dadurch herstellen, daß man anstelle der Verbindungen gemäß Formel (VIa) bzw. (VIb) solche der entsprechenden epoxy-propyl-substituierten Salze der Formel (VIIa) bzw. (VIIb) einsetzt. Das sich bei der Umsetzung zunächst bildende quaternäre Ammoniumhydroxid wird durch Zugabe der äquivalenten Menge einer Säure $H_nA$, wobei A das gewünschte Anion des Endproduktes und n die Wertigkeit des Anions bedeuten, in das Salz der Formel I überführt.

Die erfindungsgemäßen quaternären Ammoniumsalze sind, wie gesagt, als Mikrobiozide verwendbar. Die vielseitige Kombinationsmöglichkeit der Ausgangsverbindungen zur Herstellung der Salze der Formel I gestattet es, auf dem jeweiligen Anwendungszweck abgestimmte Mikrobiozide zu erhalten.

Die mikrobiozide Wirksamkeit der erfindungsgemäßen Salze umfaßt insbesondere Bakterien, Pilze und Algen. Die erfindungsgemäßen Salze können daher als Mikrobiozid in beispielsweise Brauch-, Prozeß- und Kühlwässern, in technischen Produkten, wie Farbdispersionen, Koalin-slurries, Bohr- und Schneidflüssigkeiten sowie zur Schleimbekämpfung in der Papierindustrie Verwendung finden.

Das bei bekannten, biozid wirksamen quaternären Ammoniumverbindungen übliche starke Schäumen läßt sich in den erfindungsgemäßen Salzen dadurch mindern, daß kürzerkettige Alkylreste, vorzugsweise Octylreste am Ammoniumstickstoff gebunden und/oder Bestandteil der $NR^5R^6$- oder $OR^4$-Gruppe sind. Dies ist überraschend, weil beispielsweise Ausgangssalze (VIa) mit $R^1$ gleich n-Octyl praktisch nicht biozid wirksam sind, daraus hergestellte erfindungsgemäße Salze aber eine hohe mikrobiozide Wirkung entfalten. Es gelingt somit, diese Mikrobiozide auch in technischen Bereichen in turbulent bewegten System, wie Kühlwasserkreisläufen, einzusetzen.

Wie aus den Beispielen folgt, weisen die erfindungsgemäßen Salze im Vergleich zu ihren Ausgangsverbindungen gegenüber einzelnen Mikroorganismen eine signifikant höhere mikrobiozide Wirkung auf.

Die erfindungsgemäßen Salze sind im allgemeinen nicht korrosiv. Sie lassen sich im allgemeinen auch gut biologisch abbauen.

In den folgenden Beispielen werden die Herstellung erfindungsgemäßer quaternärer Ammoniumsalze sowie deren Untersuchung auf ihre mikrobiozide Wirksamkeit beschrieben.

Beispiel 1

Herstellung des Salzes gemäß Formel (I) mit $X=NR^5R^6$, worin $R^1$=n-$C_{12}H_{25}$; $R^2=R^3=CH_3$; $R^5$=H; $R^6=CH_3$ und A=$Cl^-$ bedeuten. (1-Dodecanaminium, N-[2-hydroxy-3-[[(methylamino)thioxomethyl]thio]propyl]-N,N-dimethyl-, chlorid):

Zu 320 g einer 40 gew.-%igen wäßrigen Lösung von Natrium-N-methyl-dithiocarbamat gibt man 450 g Isopropanol und tropft dann 840 g einer 40 gew.-%igen wäßrigen Lösung von 3-Chlor-2-hydroxypropyl-N,N,N-dimethyldodecylammoniumchlorid zu. Man läßt den Reaktionsansatz über Nacht stehen und trennt das sich ausbildende zweiphasige System. Die obere, i-propanol-haltige Phase wird mehrere Stunden bei 0 °C gelagert. Das oben bezeichnete Salz kristallisiert in langen, farblosen Nadeln aus. Schmelzpunkt: 72 °C.

| | | | | | |
|---|---|---|---|---|---|
| MG 413,1 | Ber. | C 55,4 | H 10,0 | N 6,8 | S 15,6 |
| $C_{19}H_{41}N_2OS_2Cl$ | Gef. | C 55,2 | H 10,0 | N 6,5 | S 15,3 |

Die oben angegebene Struktur wurde durch IR- und NMR-Messungen bestätigt.

Beispiel 2

Herstellung des Salzes gemäß Formel (I) mit X=$NR^5R^6$, worin $R^1$=n-$C_{12}H_{25}$; $R^2$=$R^3$=$CH_3$; $R^5$=$R^6$=n-$C_8H_{17}$; A=Cl bedeuten. (1-Dodecanaminium, N-[2-hydroxy-3-[[(di-octylamino) thioxomethyl]thio]propyl]-N,N-dimethyl-, chlorid):

Zu 1.130 g einer 30 gew.-%igen Lösung von Natrium-N,N-dioctyldithiocarbamat in einer 1:1 Mischung aus Wasser/Isopropanol werden 840 g einer 40,7 gew.-%igen wäßrigen Lösung von 3-Chlor-2-hydroxy-propyl-N,N,N-dimethyldodecylammoniumchlorid zugetropft. Der sich abscheidende ölige Niederschlag wird von der kochsalzhaltigen wäßrigen Phase getrennt, mit Wasser gewaschen und im Vakuum getrocknet. Hochviskoses, schwach gelbliches Öl, praktisch geruchlos.

| | | | | | |
|---|---|---|---|---|---|
| MG 463,5 | Ber. | C 65,5 | H 11,5 | N 4,5 | S 10,2 |
| $C_{34}H_{71}N_2OS_2Cl$ | Gef. | C 64,9 | H 11,5 | N 4,3 | S 10,0 |

Beispiel 3

Herstellung des Salzes der Formel (I) mit X=$NR^4R^5$, worin $R^1$=n-$C_8H_{17}$; $R^2$=$R^3$=$CH_3$; $R^5$=H; $R^6$=n-$C_8H_{17}$; A=Cl bedeuten. (1-Octanaminium, N-[2-hydroxy-3-[[(octylamino)thioxomethyl] thio]propyl]-N,N-dimethyl-, chlorid):

Zu 1.791 g einer 12 gew.-%igen wäßrigen Lösung von Natrium-N-octyl-dithiocarbamat werden 530 g einer 54 gew.-%igen wäßrigen Lösung von 3-Chlor-2hydroxypropyl-N,N,N-dimethyloctylammoniumchlorid zugegeben. Das gewünschte Salz fällt als Feststoff sofort aus, wird abgesaugt, mit Wasser gewaschen und im Vakuum getrocknet. Eine weitere Reinigung kann durch Umfällen oder Umkristallisation aus Alkohol erfolgen.

| | | | | | |
|---|---|---|---|---|---|
| MG 455 | Ber. | C 58,0 | H 10,3 | N 6,1 | S 14,0 |
| $C_{22}H_{47}N_2OS_2Cl$ | Gef. | C 57,5 | H 10,3 | N 5,8 | S 14,4 |

Beispiel 4

Herstellung des Salzes der Formel (I) mit X=$NR^5R^6$, worin $R^1$=$R^5$=$R^6$=n-$C_8H_{17}$; $R^2$=$R^3$=$CH_3$; A=Cl bedeuten. (1-Octanaminium, N-[2-hydroxy-3-[[(di-octylamino)thioxomethyl]thio]propyl]-N,N-dimethyl-, chlorid):

Zu 1.130 g einer 30 gew.-%igen Lösung von Natrium-N,N-dioctyldithiocarbamat in einer $H_2O$/i-Propanol-Mischung werden 530 g einer 54 gew.-%igen wäßrigen Lösung von 3-Chlor-2-hydroxypropyl-N,N,N-dimethyloctylammoniumchlorid gegeben. Beim Stehenlassen bilden sich sich 2 Phasen aus, die obere, organische Phase wird abgetrennt, mit Wasser gewaschen, vom iso-Propanol befreit und getrocknet. Viskoses, gelbes Öl.

| | | | | | |
|---|---|---|---|---|---|
| MG 567,4 | Ber. | C 63,5 | H 11,2 | N 4,9 | S 11,3 |
| $C_{30}H_{63}N_2OS_2Cl$ | Gef. | C 62,8 | H 11,4 | N 4,7 | S 10,9 |

Beispiel 5

Herstellung des Salzes der Formel (I) mit X=$OR^4$, worin $R^1$=$R^4$=n-$C_8H_{17}$; $R^2$=$R^3$=$CH_3$ und A=Cl bedeuten. (1-Octanaminium, N-[2-hydroxy-3-[[(octyloxy)thioxomethyl]thio]propyl]-N,N-dimethyl-, chlorid):

244 g Kalium-dithiokohlensäure-O-n-octylester werden in 400 ml Wasser gelöst und 530 g einer 54 gew.-%igen Lösung von 3-Chlor-2-hydroxypropyl-N,N,N-dimethyloctylammoniumchlorid zugegeben. Das gewünschte Salz scheidet sich als gelbes Öl ab und wird durch Zugabe von Chloroform gelöst. Das 2-phasige System wird getrennt, die Chloroformphase sorgfältig mit Wasser gewaschen und eingeengt. Trocknen im Va-

kuum.

| | | | | | |
|---|---|---|---|---|---|
| MG 456 | Ber. | C 57,9 | H 3,0 | N 10,0 | S 14,0 |
| $C_{22}H_{46}NO_2S_2Cl$ | Gef. | C 56,9 | H 3,1 | N 10,2 | S 13,2 |

Beispiel 6

Herstellung des Salzes der Formel (I) mit X=Strukturelement (IV), wobei $R^1$=n-$C_8H_{17}$; $R^2$=$R^3$=$CH_3$; $R^7$=H; Y= Hexamethylen und A=Cl bedeuten. (4,15-Dithia-6,13-diazaoctadecane-1,18-diaminium, 2,17-dihydroxy-N,N,N',N'-tetramethyl-N,N'-dioctyl-5,14-dithioxo-, dichlorid):

Zu 2.080 g einer 15 gew.-%igen wäßrigen Lösung von Di-Natrium-hexamethylenbisdithiocarbamat werden 1060 g einer 54 gew.-%igen wäßrigen Lösung von 3-Chlor-2-hydroxypropyl-N,N,N-dimethyloctylammoniumchlorid gegeben. Das gebildete quaternäre Salz scheidet sich unmittelbar als viskoses gelbes Öl ab. Man trennt die Phasen, wäscht sorgfältig mit Wasser chlorfrei und trocknet.

| | | | | | |
|---|---|---|---|---|---|
| MG 767 | Ber. | C 53,2 | H 9,4 | N 7,3 | S 16,6 |
| $C_{34}H_{72}N_4O_2S_4Cl_2$ | Gef. | C 52,7 | H 9,6 | N 6,8 | S 16,0 |

Beispiel 7

Herstellung des Salzes gemäß Formel

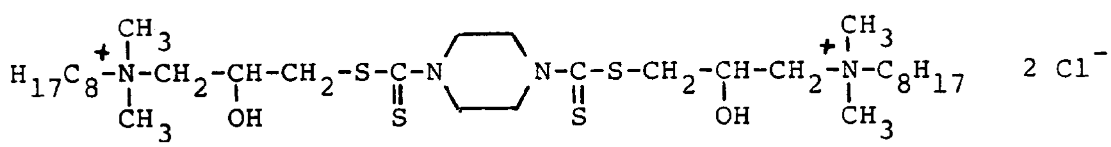

Zu 1.880 g einer 15 gew.-%igen wäßrigen Lösung von Di-Na-1,4-Piperazindiylbis(dithiocarbonat) werden 1.060 g einer 54 gew.-%-igen wäßrigen Lösung von 3-Chlor-2-hydroxy-propyl-N,N,N-dimethyloctylammoniumchlorid gegeben. Das ölig abgeschiedene quaternäre Salz wird abgetrennt und sorgfältig mit Wasser chloridfrei gewaschen. Man reinigt durch Behandeln mit Alkohol. Gelbes, viskoses Öl.

| | | | | | |
|---|---|---|---|---|---|
| MG 737 | Ber. | C 52,1 | H 8,9 | N 7,6 | S 11,4 |
| $C_{32}H_{66}N_4O_2S_4Cl_2$ | Gef. | C 51,6 | H 9,2 | N 6,9 | S 16,5 |

Beispiel 8

Bestimmung der mikrobioziden Wirkung im Time-Kill-Test.

Bei diesem in Anlehnung an die Empfehlungen des American Petroleum Instituts (API, RP 38 2nd ed., Dec. 1965) durchgeführten Test wird eine hoch angereicherte Keimsuspension (Keimzahl $10^6$-$10^8$) mit der gewünschten Menge Biozid versetzt und 24 h bei 25 °C bebrütet. Anschließend wird eine geometrische Verdünnungsreihe bis 6 durchgeführt; je 1 ml wird mit 10 ml Nähragar auf Platten vermischt und 48 h bei 37 °C bebrütet. Aus der Auszählung der Kolonien wird die Abtötungsrate berechnet.

$$\text{Abtötungsrate in \%} = 100 - \frac{\text{Keimzahl der Probe} \cdot 100}{\text{Keimzahl der Nullprobe}}$$

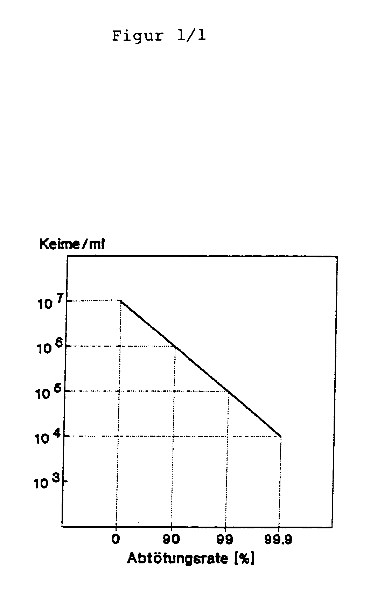

Die Figur zeigt das Verhältnis von Keimzahl zur Abtötungsrate. Hieraus wird deutlich, daß auch eine von 99,9 % auf 100 % gesteigerte Abtötungsrate einer bedeutenden Wirkungsverbesserung entspricht.

Der Tabelle ist die biozide Wirksamkeit der untersuchten Salze der Formel (I) gegen Pilze und Bakterien im Vergleich zur bioziden Wirksamkeit der Ausgangsverbindungen zu entnehmen. Angegeben ist die Abtötungsrate gegenüber den Testkeimen, wobei die Anwendungskonzentration der Biozide 50 ppm bzw. 25 ppm betrug. Die Biozide wurden als 1 gew.-%ige wäßrige Lösung der angereicherten Keimsuspension (40 ml), welche 8,5 g NaCl/l enthielt, zugesetzt.

Tabelle

| | Aspergillus niger 50 ppm | Escherichia coli 25 ppm | Staphylococcus aureus 25 ppm |
|---|---|---|---|
| N-Methylcarbamat *) | 28,3 | 8,7 | 30,4 |
| Quab R 342 | 91,0 | 99,9 | 99,9 |
| Salz gem. Beispiel 1 | 99,9 | 100 | 100 |
| Dioctylcarbamat **) | 91,9 | 99,0 | |
| Quab R 342 | 91,0 | 99,9 | 99,5 |
| Salz gem. Beispiel 2 | 91,3 | 99,9 | 99,9 |
| Octylcarbamat ***) | 6,7 | 93,1 | 95,2 |
| Quab R 286 | 0 | 23,2 | 0 |
| Salz gem. Beispiel 3 | 99,9 | 100 | 100 |
| Dioctylcarbamat **) | 91,9 | 99,0 | |
| Quab R 286 | 0 | 23,2 | 0 |
| Salz gem. Beispiel 4 | 90,7 | 88,8 | 100 |
| O-Octyldithio-kohlensäure-Na-Salz | 6,7 | 94,1 | 99,0 |
| Quab R 286 | 0 | 23,2 | 0 |
| Salz gem. Beispiel 5 | 86,4 | 50,0 | 99,0 |
| Hexamethylendiyl-bis(dithiocarbamin-säure-Na-Salz | 13,3 | 12,3 | 60,0 |
| Quab R 286 | 0 | 23,2 | 0 |
| Salz gem. Beispiel 6 | 98,9 | 99,9 | 100 |
| 1,4-Piperazindiyl-bis(dithiocarbonat) | 0 | 46,2 | 21,7 |
| Quab R 286 | 0 | 23,2 | 0 |
| Salz gem. Beispiel 7 | 92,7 | 99,9 | 99,8 |

*) N-Methyldithiocarbaminsäure Natriumsalz
**) N,N-Dioctyldithiocarbaminsäure Natriumsalz
***) N,-Octyldithiocarbaminsäure Natriumsalz

Quab R 342 (Degussa AG) = 3-Chlor-2-hydroxypropyl-N,N-dimethyldodecylammoniumchlorid

Quab R 286 (Degussa AG) = 3-Chlor-2-hydroxypropyl-N,N-dimethyloctylammoniumchlorid

## Patentansprüche

1. Quaternäre Ammoniumsalze der allgemeinen Formel (I)

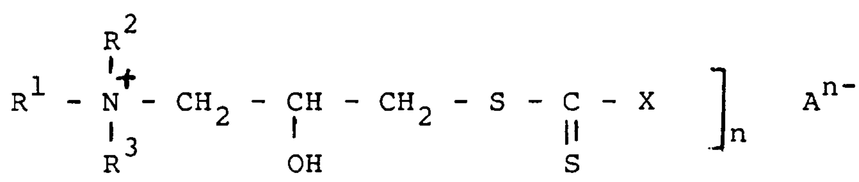

(I),

worin bedeuten:

$R^1$ eine lineare ($C_8$-$C_{16}$)-Alkylgruppe oder eine ($C_8$-$C_{16}$)-Alkylphenoxyethoxyethylgruppe, wenn $R^2$ und $R^3$ gleich sind und Methyl oder Hydroxyethyl bedeuten, oder ein Strukturelement der allgemeinen Formel (II)

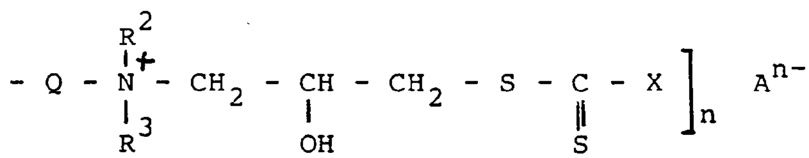

(II),

worin Q für α, ω-($C_2$-$C_6$)-Alkylen, das innenständig auch methylsubstituiert sein kann,

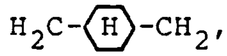

$CH_2$-$CH_2$-O-$CH_2$-$CH_2$ oder $CH_2$-$CH_2$-N($CH_3$ )-$CH_2$-$CH_2$ steht, wenn $R^2$ und $R^3$ in Formel (I) und (II) jeweils Methyl bedeuten oder im Falle von Q gleich Ethylen $R^2$ in (I) und (II) Methyl und $R^3$ in (I) und (II) auch gemeinsam für eine Ethylengruppe stehen können;

X die Gruppe $OR^4$, worin $R^4$ für einen linearen oder verzweigten Alkylrest mit 8 bis 18 C-Atomen steht, oder
die Gruppe $NR^5R^6$, worin $R^5$ Wasserstoff oder eine lineare ($C_8$-$C_{18}$)-Alkylgruppe und $R^6$ eine ($C_1$-$C_{18}$)-Alkyl- oder Benzylgruppe bedeuten oder $NR^5R^6$ für den Pyrrolidino-, 1,3-Oxazolidino, Piperidino oder Morpholinorest steht, wobei in das Strukturelement (II) enthaltenden Salzen die Gruppe $NR^5R^6$ mindestens einen linearen ($C_8$-$C_{12}$)-Alkylrest aufweist, oder
ein Strukturelement der allgemeinen Formel (III) oder (IV)

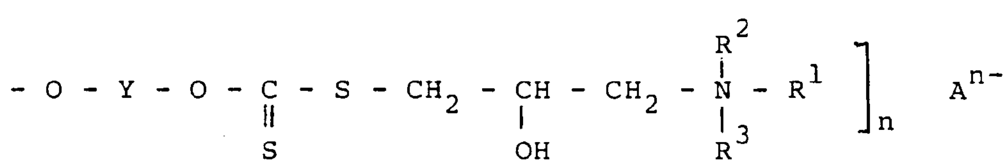

(III)

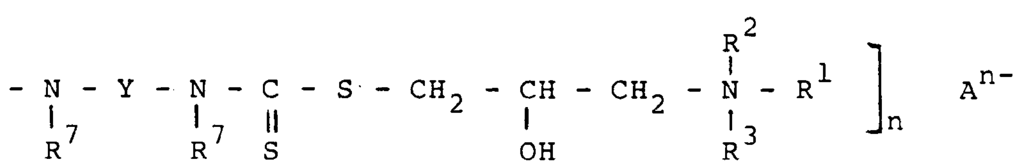

(IV),

worin Y die gleiche Bedeutung wie Q hat und $R^7$ Wasserstoff oder Methyl bedeutet, jedoch im Falle von Y gleich Ethylen beide Substituenten $R^7$ auch gemeinsam für eine Ethylengruppe stehen können, und wobei in das Strukturelement (III) oder (IV) enthaltenden Salzen $R^1$ nicht dem Strukturelement (II) entspricht;

A ein Halogenid, Sulfat, Hydrogensulfat, Hydrogen- oder Dihydrogenphosphat, Nitrat, Formiat, Acetat, Benzoat und

n die Wertigkeit 1 oder 2 des Anions.

**2.** Quaternäre Ammoniumsalze nach Anspruch 1,
dadurch gekennzeichnet,
daß $R^1$ eine lineare ($C_8$-$C_{12}$)-Alkyl-, $R^2$ und $R^3$ eine Methylgruppe, $R^5$ Wasserstoff oder eine lineare ($C_8$-$C_{12}$)-Alkyl- und $R^6$ eine Methyl- oder eine lineare ($C_8$-$C_{12}$)-Alkylgruppe bedeuten.

**3.** Quaternäre Ammoniumsalze nach Anspruch 1,
dadurch gekennzeichnet,
daß Y 1,6-Hexamethylen, $R^7$ Wasserstoff, $R^1$ eine lineare ($C_8$-$C_{12}$)-Alkylgruppe und $R^2$ und $R^3$ Methylgruppen bedeuten.

**4.** Quaternäre Ammoniumsalze nach Anspruch 1,
dadurch gekennzeichnet,
daß $R^1$ eine lineare ($C_8$-$C_{12}$)-Alkyl-, $R^2$ und $R^3$ Methyl- und $R^4$ eine lineare ($C_8$-$C_{12}$)-Alkylgruppe bedeuten.

**5.** Quaternäre Ammoniumsalze nach Anspruch 1,
dadurch gekennzeichnet,
daß A Chlorid bedeutet.

**6.** Verfahren zur Herstellung quaternärer Ammoniumsalze gemäß allgemeiner Formel (I) des Anspruchs 1,
dadurch gekennzeichnet,
daß man in wäßriger Lösung ein Alkalisalz einer Dithiocarbaminsäure der allgemeinen Formel (Va) oder (Vb)

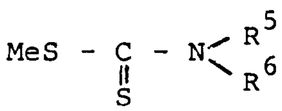 (Va) 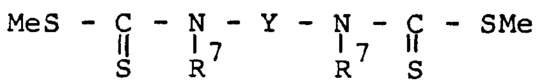 (Vb)

oder ein Alkalisalz eines Dithiokohlensäure-O-esters der allgemeinen Formel (Vc) oder (Vd)

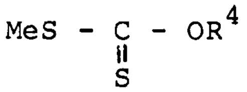 (Vc) 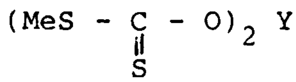 (Vd),

worin Me Li, Na oder K sein kann und $R^4$, $R^5$, $R^6$, $R^7$ und Y die Bedeutung gemäß Anspruch 1 haben, mit einem 3-Halogen-2-hydroxypropyltrialkylammoniumsalz der allgemeinen Formel (VIa) oder (VIb)

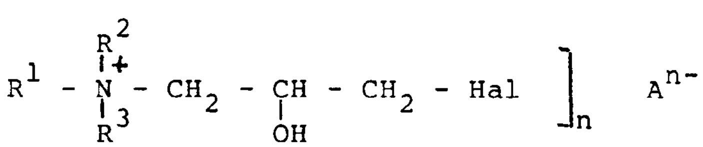 (VIa)

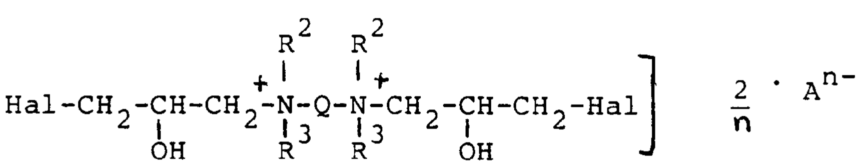 (VIb),

worin Hal Chlor oder Brom und $R^1$, $R^2$, $R^3$ A, n und Q die Bedeutung gemäß Anspruch 1 haben, umsetzt und das gebildete Salz der Formel (I) in bekannter Weise aus dem Reaktionsgemisch isoliert und, falls erwünscht, reinigt.

**7.** Verfahren zur Herstellung quaternärer Ammoniumsalze gemäß allgemeiner Formel (I) des Anspruchs 1,
dadurch gekennzeichnet,
daß man in wäßriger Lösung ein Alkalisalz einer Dithiocarbaminsäure der allgemeinen Formel (Va) oder

(Vb)

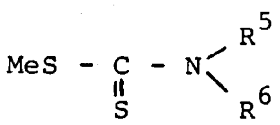 (Va) 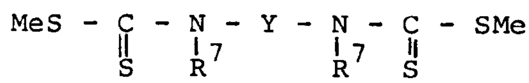 (Vb)

oder ein Alkalisalz eines Dithiokohlensäure-O-esters der allgemeinen Formel (Vc) oder (Vd)

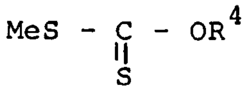 (Vc) 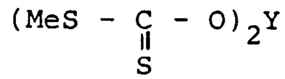 (Vd),

worin Me Li, Na oder K sein kann und $R^4$, $R^5$, $R^6$, $R^7$ und Y die Bedeutung gemäß Anspruch 1 haben, mit einem 2,3-Epoxy-propyl -trialkylammoniumsalz der allgemeinen Formel (VIIa) oder (VIIb)

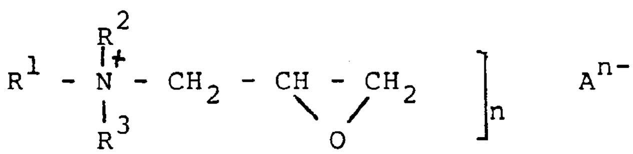 (VIIa)

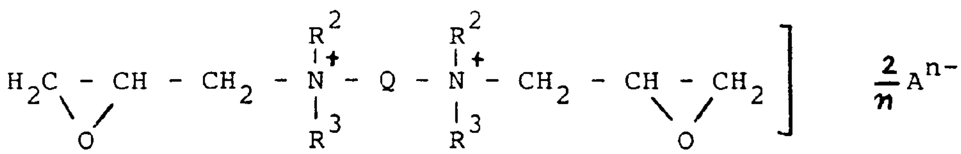 (VIIb),

worin $R^1$, $R^2$, $R^3$, A, n und Q die Bedeutung gemäß Anspruch 1 haben, umsetzt und das sich bildende quaternäre Ammoniumhydroxid durch Zugabe einer äquivalenten Menge einer Säure $H_nA$, wobei A die Bedeutung gemäß Anspruch 1 hat, in das Salz der Formel I überführt.

8. Verwendung der quaternären Ammoniumsalze gemäß Formel I des Anspruchs 1 als Mikrobiozid.

## Claims

1. Quaternary ammonium salts of the general formula (I)

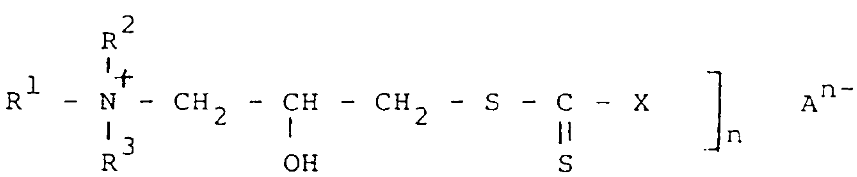 (I)

wherein:

$R^1$ represents a linear ($C_8$-$C_{16}$) -alkyl group or a ($C_8$-$C_{16}$) -alkylphenoxyethoxyethyl group, when $R^2$ and $R^3$ are identical and represent methyl or hydroxyethyl, or a structural element of the general formula (II)

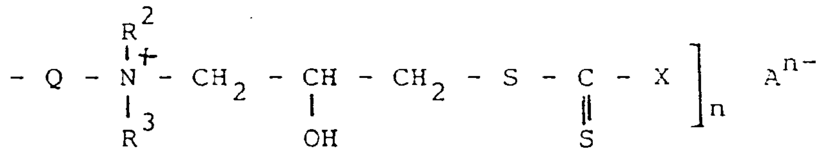

(II),

wherein Q stands for α,ω-($C_2$-$C_6$)-alkylene, which may also be methyl-substituted in an intermediate position,

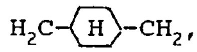

$CH_2$-$CH_2$-O-$CH_2$-$CH_2$ or $CH_2$-$CH_2$-N($CH_3$)-$CH_2$-$CH_2$ when $R^2$ and $R^3$ in each case represent methyl in formula (I) and (II) or in the case of Q being equal to ethylene $R^2$ may stand for methyl in (I) and (II) and $R^3$ in (I) and (II) may together stand for one ethylene group;

X represents the group $OR^4$, wherein $R^4$ stands for a linear or branched alkyl radical having from 8 to 18 C atoms, or
the group $NR^5R^6$, wherein $R^5$ represents hydrogen or a linear ($C_8$-$C_{18}$) -alkyl group and $R^6$ represents a ($C_1$-$C_{18}$) -alkyl or benzyl group or $NR^5R^6$ stands for the pyrrolidino, 1, 3-oxazolidino, piperidino or morpholino radical, wherein in salts containing the structural element (II) the group $NR^5R^6$ has at least one linear ($C_8$-$C_{12}$) -alkyl radical, or
a structural element of the general formula (III) or (IV)

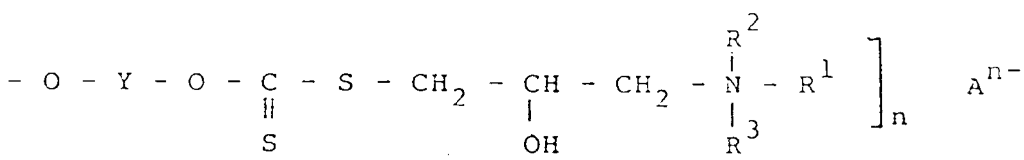

(III)

(IV)

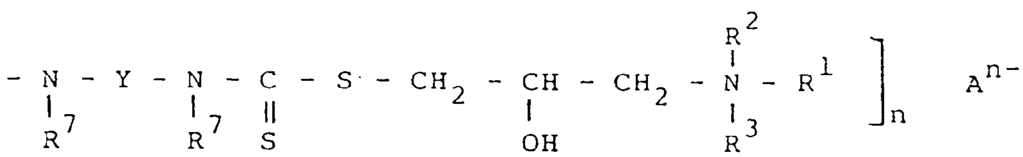

wherein Y has the same meaning as Q and $R^7$ represents hydrogen or methyl, but if Y is ethylene both the substituents $R^7$ may also together stand for one ethylene group, and wherein in salts containing the structural element (III) or (IV) $R^1$ does not correspond to the structural element (II);

A represents a halide, sulphate, hydrogen sulphate, hydrogen phosphate or dihydrogen phosphate, nitrate, formate, acetate, benzoate, and

n represents the valency 1 or 2 of the anion.

**2.** Quaternary ammonium salts according to Claim 1, characterized in that $R^1$ represents a linear ($C_8$-$C_{12}$) -alkyl group, $R^2$ and $R^3$ represent a methyl group, $R^5$ represents hydrogen or a linear ($C_8$-$C_{12}$) -alkyl group and $R^6$ represents methyl or a linear ($C_8$-$C_{12}$) -alkyl group.

**3.** Quaternary ammonium salts according to Claim 1, characterized in that Y represents 1,6-hexamethylene, $R^7$ represents hydrogen, $R^1$represents a linear ($C_8$-$C_{12}$)-alkyl group and $R^2$ and $R^3$ represent methyl groups.

**4.** Quaternary ammonium salts according to Claim 1, characterized in that $R^1$ represents a linear ($C_8$-$C_{12}$)-alkyl group, $R^2$ and $R^3$ represent methyl groups and $R^4$ represents a linear ($C_8$-$C_{12}$)-alkyl group.

**5.** Quaternary ammonium salts according to Claim 1, characterized in that A represents chloride.

6. Process for the preparation of quaternary ammonium salts according to general formula (I) of Claim 1, characterized in that in an aqueous solution, an alkali salt of a dithiocarbamic acid of the general formula (Va) or (Vb)

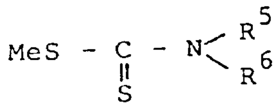 (Va) 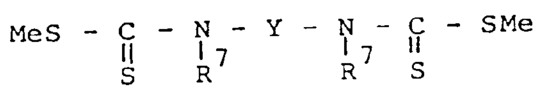 (Vb)

or an alkali salt of a dithiocarbonic acid O-ester of the general formula (Vc) or (Vd)

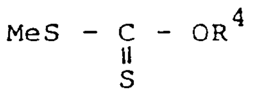 (Vc) 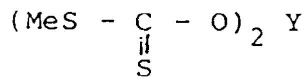 (Vd),

wherein Me can be Li, Na or K and $R^4$, $R^5$, $R^6$, $R^7$ and Y have the meanings as in Claim 1, is reacted with a 3-halo-2-hydroxypropyltrialkyl ammonium salt of the general formula (VIa) or (VIb)

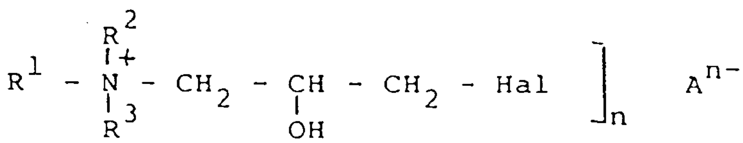 (VIa)

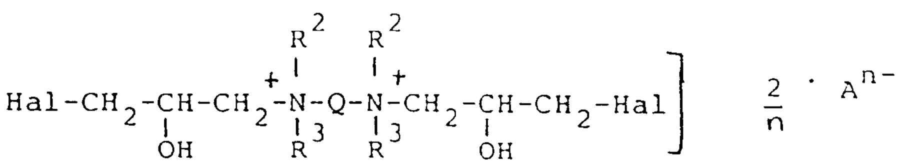 (VIb),

wherein Hal is chlorine or bromine and $R_1$, $R_2$, $R_3$ have the meanings as in Claim 1, and the salt formed of the formula (I) is isolated from the reaction mixture in known manner and, optionally , is purified.

7. Process for the preparation of quaternary ammonium salts according to general formula (I) in Claim 1, characterized in that in an aqueous solution, an alkali salt of a dithiocarbamic acid of the general formula (Va) or (Vb)

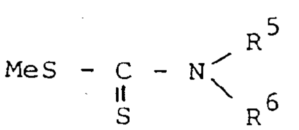 (Va) 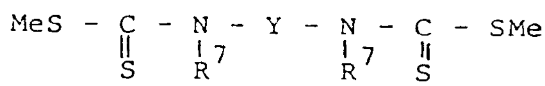 (Vb)

or an alkali salt of a dithiocarbonic acid O-ester of the general formula (Vc) or (Vd)

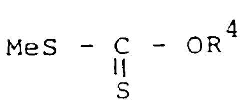 (Vc) 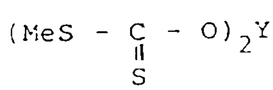 (Vd),

wherein Me can be Li, Na or K and $R^4$, $R^5$, $R^6$, $R^7$ and Y have the meanings as in Claim 1, is reacted with a 2,3-epoxypropyl-trialkyl ammonium salt of the general formula (VIIa) or (VIIb)

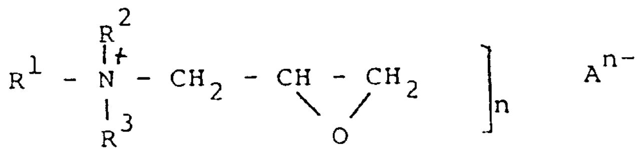 (VIIa)

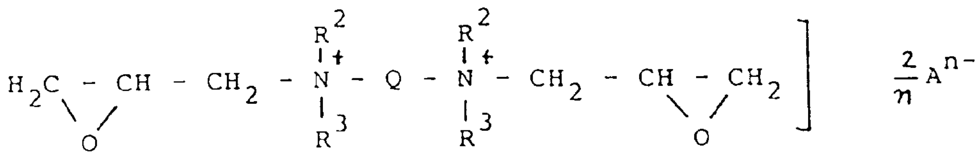 (VIIb),

wherein $R^1$, $R^2$, $R^3$, A, n and Q have the same meanings as in Claim 1, and the quaternary ammonium hydroxide formed is converted, by the addition of an equivalent quantity of an acid $H_nA$, in which A has the meaning according to Claim 1, to the salt of the formula I.

8. Use of the quaternary ammonium salts according to formula I of Claim 1 as a microbiocide.

**Revendications**

1. Composés d'ammonium quaternaire de formule générale (I) :

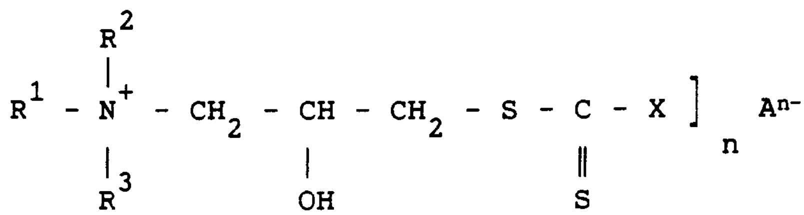 (I)

dans laquelle :

$R^1$ représente un groupe alkyle linéaire ($C_6$-$C_{16}$) ou un groupe alkylphénoxyéthoxyéthyle, quand $R^2$ et $R^3$ sont identiques et un méthyle ou hydroxyéthyle, ou un élément structural de formule générale (II) :

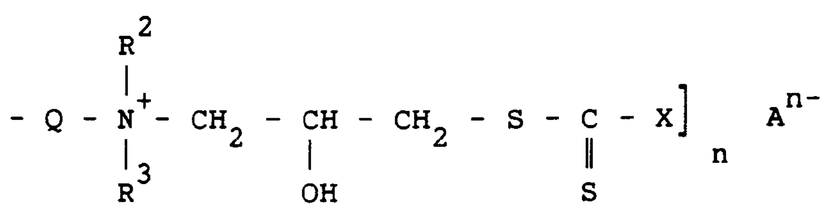 (II)

dans lequel Q représente un $\alpha,\omega$ -alcylène ($C_2$-$C_6$), qui peut aussi être intérieurement méthylsubstitué, $H_2C$- H -$CH_2$, $CH_2$-O-$CH_2$-$CH_2$ ou $CH_2$-$CH_2$-N($CH_3$)-$CH_2$-$CH_2$, quand $R^2$ et $R^3$ représentent chacun dans les formules (I) et (II) un méthyle, ou dans le cas où Q est identique à l'éthylène, $R^2$ dans (I) et (II) peut être le méthyle et $R^3$ dans (I) et (II) peut être aussi un groupe éthylène,

X représente le groupe $OR^4$, dans lequel $R^4$ est un reste alkyle linéaire ou ramifié de 8 à 18 atomes de C,
ou le groupe $NR^5R^6$, dans lequel $R^5$ est l'hydrogène ou un groupe alkyle ($C_8$-$C_{18}$) linéaire et $R^6$ est un groupe alkyle $C_1$-$C_{18}$ ou benzyle ou $NR^5R^6$ représente le reste pyrrolidino-, 1,3-oxazolidino, pipéridino ou morpholino, dans des sels contenant l'élément structural (II) le groupe $NR^5R^6$ présentant au moins un reste alkyle ($C_8C_{12}$) linéaire, ou un élément structural de formule générale (III) ou (IV) :

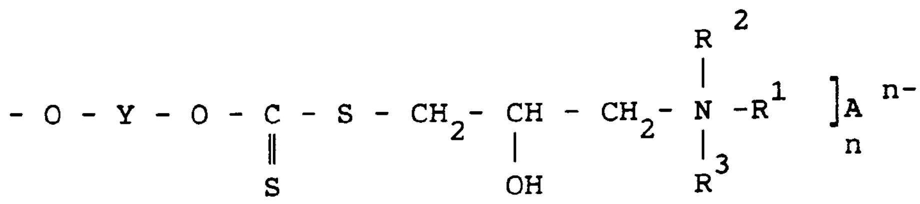

(III)

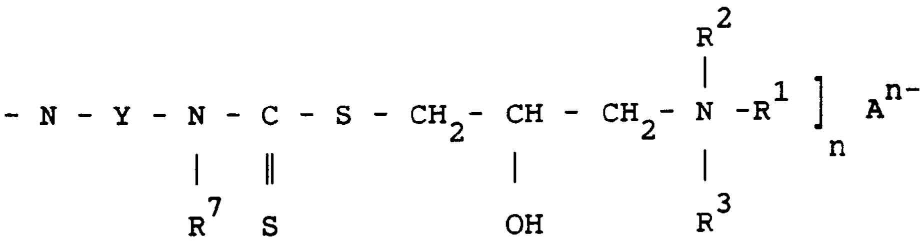

(IV)

dans lesquelles Y a la même signification que Q et $R^7$ représente l'hydrogène ou le méthyle, cependant dans le cas ou Y est l'éthylène, les deux substituants $R^7$ peuvent aussi représenter un groupe éthylène, et lorsque dans des sels contenant l'élément structural (III) ou (IV), $R^1$ ne correspond pas à l'élément structural (II) ;

A est un halogénure, sulfate, hydrogénosulfate, hydrogéno- ou dihydrogénophosphate, nitrate, formiate, acétate, benzoate et

n est la valence 1 ou 2 de l'anion.

2. Sels d'ammonium quaternaires selon la revendication 1, caractérisés en ce que $R^1$ représente un alkyle ($C_8$-$C_{12}$) linéaire, $R^2$ et $R^3$ un groupe méthyle, $R^5$ l'hydrogène ou un alkyle ($C_8$-$C_{12}$) linéaire et $R^6$ un méthyle ou un groupe alkyle linéaire ($C_8$-$C_{12}$).

3. Sels d'ammonium quaternaires selon la revendication 1, caractérisés en ce que Y représente le 1,6-hexaméthylène, $R^7$ l'hydrogène, $R^1$ un groupe alkyle ($C_8$-$C_{12}$) linéaire et $R^2$ et $R^3$ des groupes méthyles.

4. Sels d'ammonium quaternaires selon la revendication 1, caractérisés en ce que R1 représente un alkyle ($C_8$-$C_{12}$) linéaire, $R^2$ et $R^3$ un méthyle et $R^4$ un groupe alkyle ($C_8$-$C_{12}$) linéaire.

5. Sels d'ammonium quaternaires selon la revendication 1, caractérisés en ce que A représente un chlorure.

6. Procédé de préparation des sels d'ammonium quaternaires selon la formule générale (I) de la revendication 1, caractérisé en ce qu'on fait réagir en solution aqueuse un sel alcalin d'un acide dithiocarbamique de formule générale (Va) ou (Vb) :

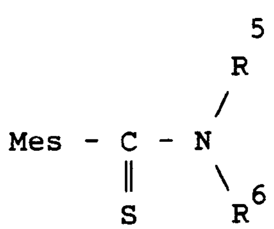

(Va)

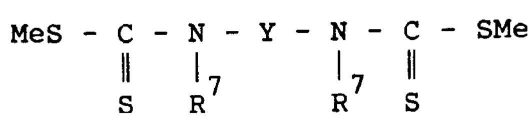

(Vb)

ou un sel alcalin d'un O-ester d'acide dithiocarbonique de formule générale (Vc) ou (Vd) :

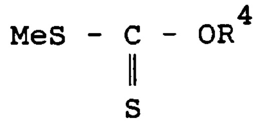 (Vc) 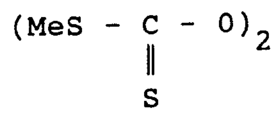 (Vd)

dans lesquelles Me peut être Li, Na ou K et $R^4$, $R^5$, $R^6$, $R^7$ et Y ont la signification donnée pour les substances de l'invention avec un sel de 3-halogéno -2-hydroxypropyltrialkylammonium de formule générale (VIa) ou (VIb) :

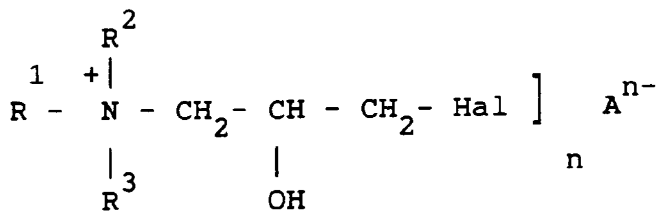 (VIa)

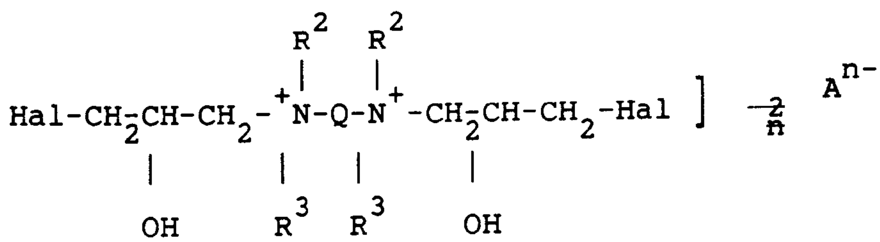 (VIb)

dans lesquelles Hal est le chlore ou le brome et $R^1$, $R^2$, $R^3$, A, n et Q ont la signification selon la revendication 1 et on isole le sel formé de formule (I) du mélange réactionnel de manière connue et si c'est souhaité on purifie.

7. Procédé de préparation des sels d'ammonium quaternaires selon la formule générale (I) de la revendication 1, caractérisé en ce qu'on fait réagair en solution aqueuse un sel alcalin d'un acide dithiocarbamique de formule générale (Va) ou Vb) :

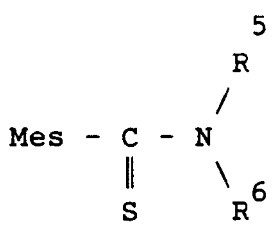 (Va)

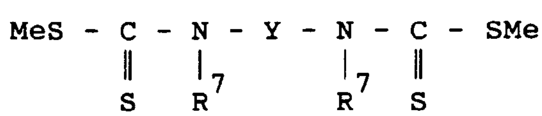 (Vb)

ou un sel alcalin d'un O-ester d'acide dithiocarbonique de formule générale (Vc) ou (Vd) :

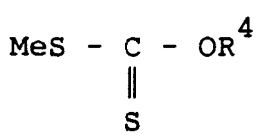 (Vc) 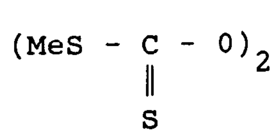 (Vd)

dans lesquelles Me peut être Li, Na ou K et $R^4$, $R^5$, $R^6$, $R^7$ et Y ont la signification selon la revendication 1, avec un sel 2,3-époxypropyltrialkylammonium de formule générale (VIIa) ou (VIIb) :

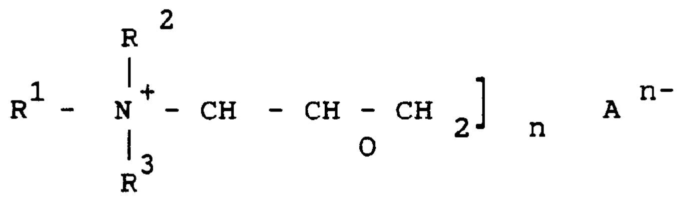

(VIIa)

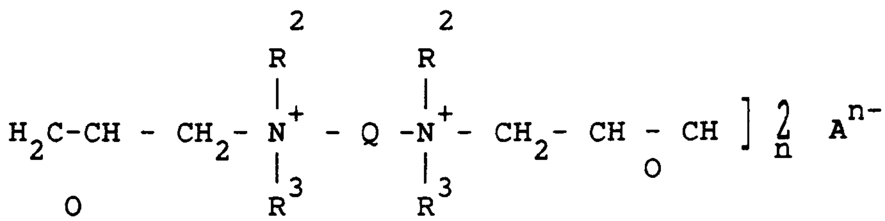

(VIIb)

dans lesquelles $R^1$, $R^2$, $R^3$, A, n et Q ont la signification selon la revendication 1 et on transforme l'hydroxyde d'ammonium quaternaire qui se forme par addition d'une quantité équivalente d'un acide $H_nA$, en sel de formule (I), A ayant la signification selon la formule (I).

8. Utilisation des sels d'ammonium quaternaire selon la formule (I) de la revendication 1 comme microbiocides.

Figur 1/1